Europäisches Patentamt
European Patent Office
Office européen des brevets

(19)

(11) Publication number : 0 457 570 A1

# EUROPEAN PATENT APPLICATION

(12)

(21) Application number : 91304363.4

(22) Date of filing : 15.05.91

(51) Int. Cl.[5] : C07H 19/10, C07H 19/073, A61K 31/70, A61K 47/48, C12P 19/30

(30) Priority : 16.05.90 JP 126278/90

(43) Date of publication of application : 21.11.91 Bulletin 91/47

(84) Designated Contracting States : AT BE CH DE DK ES FR GB GR IT LI LU NL SE

(71) Applicant : TOYO JOZO KABUSHIKI KAISHA
632-1 Mifuku Ohito-cho
Tagata-gun Shizuoka-ken (JP)

(72) Inventor : Shuto, Satoshi
690-10, Makinokou, Shuzenji-cho
Tagata-gun, Shizuoka (JP)
Inventor : Itoh, Hiromichi
38-2, Sankeidai
Mishima-shi, Shizuoka (JP)
Inventor : Obara, Takumi
59-1, Ohdoi, Kannami-cho
Tagata-gun, Shizuoka (JP)
Inventor : Fujiwara, Tatsuro
310, Nirayama, Nirayama-cho
Tagata-gun, Shizuoka (JP)

(74) Representative : Woods, Geoffrey Corlett et al
J.A. KEMP & CO. 14 South Square Gray's Inn
London WC1R 5LX (GB)

(54) **Nucleoside-phospholipid conjugate.**

(57) A compound which is a nucleoside-phospholipid conjugate of formula :

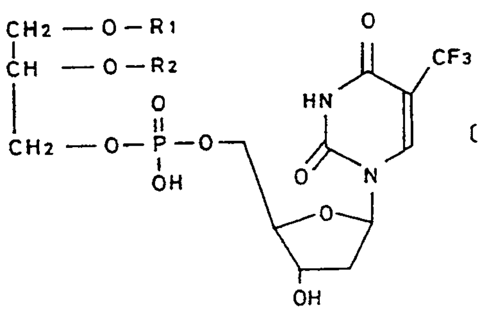

( I )

wherein $R_1$ and $R_2$, which are the same or different, are $C_{14\ 20}$ aliphatic acyl groups or a pharmacologically acceptable salt thereof.

EP 0 457 570 A1

The present invention relates to nucleoside-phospholipid conjugates and pharmacologically acceptable salts thereof.

Nucleoside antitumor agents have been widely used as effective chemotherapeutics for neoplastic cells. In their application as antitumor-chemotherapeutics, however, several problems have arisen. For example, in vivo enzymatic phosphorylation of the hydroxyl group at the 5′-position of a nucleoside anti-neoplastic agent is essential for its antitumor activity; the agent is decomposed to an inactive substance by enzymatic reactions such as phosphorolysis or deamination; the resistance of tumor cells to antitumor agents increases; and the agent is sometime toxic to normal mitotic cells. Many nucleoside derivatives have been synthesized to try to overcome the disadvantages of known nucleoside antitumor agents.

A phospholipid conjugate, a 5-fluoro-2′-deoxyuridine-5′-phosphate derivative has been reported (JP-A-61-91195).

The present inventors have found that phospholipase D effectively catalyzes the transfer reaction of the phosphatidyl residue from glycerophospholipid to the primary hydroxyl group of a nucleoside, and hence a variety of nucleoside-phospholipid conjugates including arabinoside nucleoside-phospholipid conjugates can be prepared (US-A-4,797,479).

The resistance of tumor cells to 5-fluorouracil (hereinafter referred to as 5-FU), a commonly used antitumor agent, is known. 2′-deoxy-5-trifluoromethyluridine is known to be effective against some 5-FU resistant cells and to have antitumor and antiviral activities in vivo [Heiderberger et al., Cancer Res., 24: 1979 (1964), Kaufman, H.E. et al., Science, 145: 585 (1964)]. 2′-deoxy-5-trifluoromethyluridine is, however, decomposed by thymidine phosphorylase in vivo, and is therefore not effective for clinical use. Hence 2′-deoxy-5-trifluorouridine derivatives which are effective against 5-FU resistant tumor cells and not inactivated by nucleoside phosphorylase in vivo are desired.

We have surprisingly found a nucleoside-phospholipid conjugate which has activity against 5-FU resistant tumor cells and which is not decomposed by nucleoside phosphorylase in vivo.

The present invention provides a compound which is a nucleoside-phospholipid conjugate of formula (1):

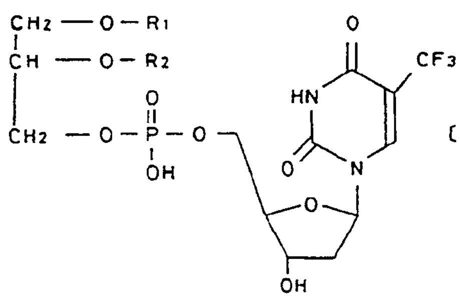

( I )

wherein $R_1$ and $R_2$, which are the same or different, are $C_{14-20}$ aliphatic acyl groups or a pharmacologically acceptable salt thereof.

$R_1$ is preferably a $C_{16-18}$ saturated or unsaturated aliphatic acyl group. $R_2$ is preferably a $C_{16-18}$ saturated or unsaturated aliphatic acyl group. For example, $R_1$ is a palmitoyl, stearoyl or oleoyl group and $R_2$ is a palmitoyl, stearoyl, oleoyl or linoleoyl group. Preferred compounds are those wherein $R_1$ and $R_2$ are both palmitoyl, both oleoyl, both stearoyl, and those wherein $R_1$ is palmitoyl or stearoyl and $R_2$ is oleoyl or linoleoyl.

The compounds of the present invention may, for example, be prepared from a glycerophospholipid such as a phosphatidylcholine of formula (II):

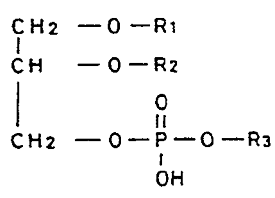

wherein $R_1$ and $R_2$ are as defined above and $R_3$ is a choline residue.

The phosphatidyl choline can be synthesized or is commercially available. An example of the nucleoside is 2′-deoxy-5-trifluoromethyluridine.

The compound of the present invention can, for example, be obtained by reacting a glycerophospholipid

derivative with a nucleoside in the presence of a phospholipase D and, if required, metallic ions in a suitable solvent. A preferred example of a phospholipase D is phospholipase D-P (hereinafter referred to as PLDP - obtainable from Toyo Jozo Co., Catalogue no. P-39) obtainable by culturing a broth of Streptomyces sp. AA586 FERM P-6100 (JP-A-58-152481).

The amount of catalyst is at least 0.1 unit phospholipase D - P per 1 mg of phosphatidyl chloline, and is preferably 1 - 100 units. Examples of suitable solvent are two-phase systems of organic solvent and aqueous solvent, for example a mixture of organic solvent such as ether, benzene, methylenechloride, ethyl acetate or chloroform and buffer solution of pH 3 - 10, preferablly pH 3 - 6. A general example of a water soluble salt for generation of metallic ion is calcium chloride. Reaction temperature is generally 10 - 60°C and reaction time is 30 minutes to 5 days. Prefered conditions are ; reaction at 25 - 45°C for 30 min. - 2 days, in 500 - 10,000 units PLDP and 0.1 - 5 m mol of phosphatidyl choline per 1 m mol of 2'- deoxy - 5 - trifluoromethyluridine, and two-phase systems of aqueous solvent of pH 4 - 7 and chloroform or benzene. The thus-obtained nucleoside-phospholipid conjugate according to the invention can be purified by a partition method and silica-gel chromatography.

One-step syntheis of nucleoside-phospholipid conjugate of the present invention is illustrated as follows:

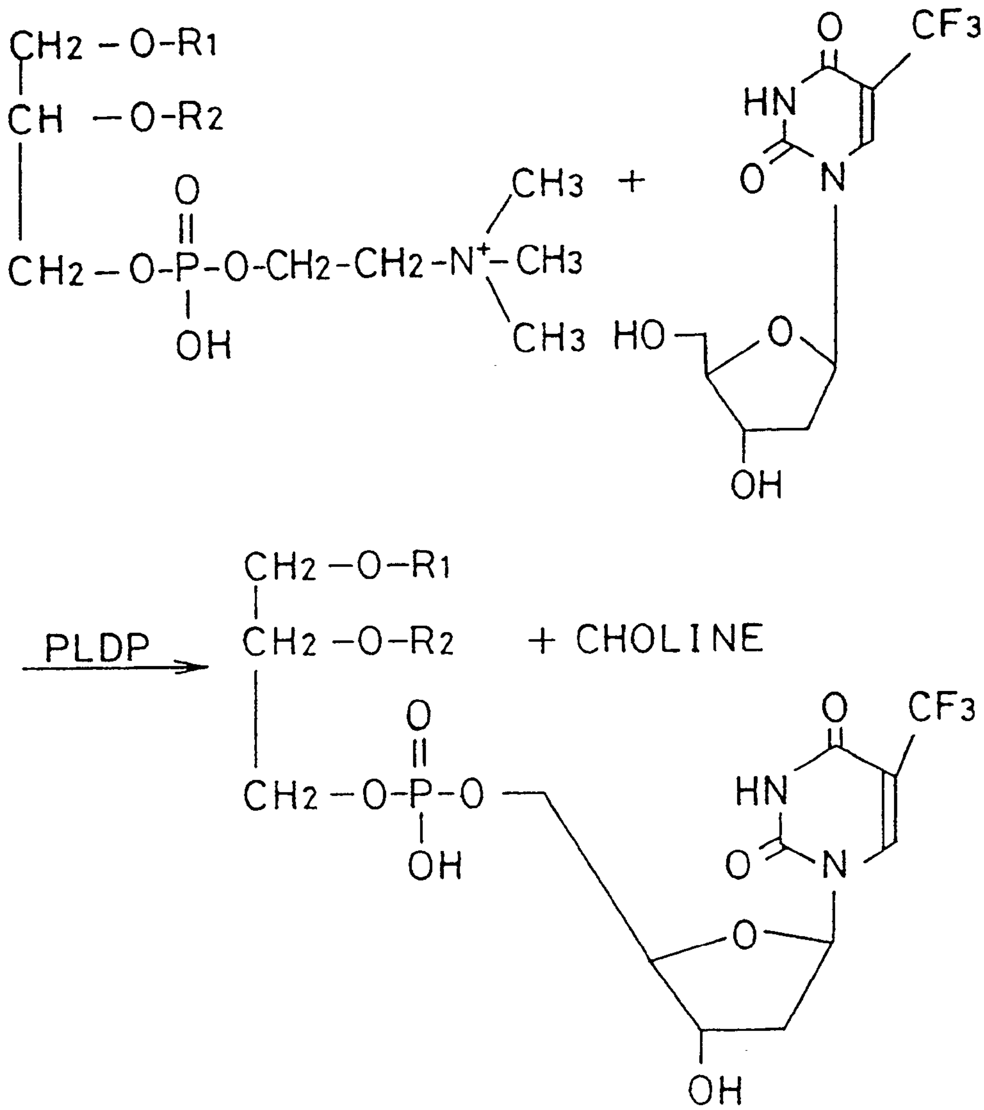


The thus-obtained nucleoside-phospholipid conjugate is a compound wherein the phosphate group in phospholipid and the primary hydroxyl group at 5' - position in 2'- deoxy - 5 - trifluoromethyluridine are bonded and is a novel compound.

The thus-obtained product can be prepared as a non-toxic, pharmacologically acceptable salt, such as sodium salt, and can be administered in general orally or in the form of suspension with sterilized distilled water.

Effect of the Invention

The thus-prepared nucleoside-phospholipid conjugate of the present invention has the advantages that: it is more lipophilic as compared with original nucleoside, 2'- deoxy - 5 - trifluoromethyluridine; it is not easily excreted, that is, it is more active for a long time; it is not affected by enzymatic inactivation reaction such as phosphorolysis with phosphorylase; it has higher affinity to cell membranes; antineoplactic nucleioside 5'-monophosphate of 2'- deoxy - 5 - trifluoromethyluridine is generated in cells without action of kinase; and it has long action and increased activity with low toxicity.

The novel nucleoside-phospholipid conjugate of the present invention reveals marked antitumor activity in vivo and in addition to cytotoxic activity against 5FU resistant cells.

Antitumor activities against Meth A fibrosarcoma and 5 - FU resistant P 388 leukemia are shown in the ensuring examples, according to the following list of specifications.

Animals:

BALB/c mice, male, age 6 weeks;
$BDF_1$ mice, male, age 5 weeks;
Charles River Japan Inc.,
5 mice in an experimental group and 7 mice in control group.

Tumor cells:

Meth A fibrosarcoma cells; 1 X $10^6$ cells/0.2 ml are inoculated subcutaneously in abdomen of BALB/c mice.

5 - FU resistant P 388 leukemia cells;

5 - FU resistant P 388 leukemia cells are established by intraperitoneally administering 20 mg/kg of 5 - FU, once a day, in P 388 leukemia cells intraperitoneally inoculated $BDF_1$ mice, and being passaged for 7 generations. The said resistant cells show 50 times resistant against 5 - FU. 1X $10^6$ cells/0.2 ml are inoculated intraperitoneally in $BDF_1$ mice.

Samples:

| Compound No | $R_1$ | $R_2$ | Nucleoside Residue |
|---|---|---|---|
| 1 | palmitoyl | palmitoyl | $THF_3Udr$ |
| 2 | stearoyl | stearoyl | 〃 |
| 3 | oleoyl | oleoyl | 〃 |
| 4 | palmitoyl | oleoyl | 〃 |
| 5 | palmitoyl | linoleoyl | 〃 |
| 6 | stearoyl | oleoyl | 〃 |
| 7 | stearoyl | linoleoyl | 〃 |
| 8 | palmitoyl | palmitoyl | FUDR |

FUDR : 2' - deoxy -5 - fuluorourdine - 5'- yl

$TF_3Udr$: 2' - deoxy - 5 - trifluoromethyluridine - 5'- yl.

Preparation of samples and administration of drugs:

Samples are suspended or dissolved in distilled water upto appropriate concentration by sonication. 0.1 ml/10g body weight is administered.

Administration: starting 24 hours after inoculation of tumor cells, once a day for 5 days orally or intraperitoneally.

Evaluation of the effect:

In Meth A fibrosarcoma inoculated mice, a volume of tumor is determined by measuring the minor axis and major axis and calculating according to the following equation.

$$\text{volume of tumor } (mm^3) = \{\text{minor axis}(mm)\}^2 \times \text{major axis}(mm)/2$$

Evaluation is determined by the value T/C (%) according to average tumor volume (C) of control group and average tumor volume (T) of treated group.

In 5 - FU resistant P 388 leukemia transplanted group, an evaluation is make according to an increase in life span ( ILS % ).

Results:

Referring to a evaluation standard for antitumor effect established in U.S. National Cancer Institute (NCI), significant effect on antitumor effect for 5 - FU resistant P 388 luekemia is set to the value ILS 25 % or over

and for Meth A fibrosarcoma is the value T/C below 40 %. As shown in Table 1, each of the nucleosidephospolipid conjugate ( compound 1 - 7 ) of the present invention illustrated in this table is administered at 0.1 m mol/kg/day for 5 succesive days, and each is shown to suppress growth of Meth A fibrosarcoma significantly within T/C 40%. In contrast, parent compound 2' - deoxy - 5 - trifluoromethyluridine shows Meth A fibrosarcoma growth inhibition as T/C 61.2% in which no significant antitumor effect is observed. A temporary little decrease in body weight is observed at 0.5 m mol/kg, single oral administration, however no death in each group is observed. Also, as shown in Table 2, the compounds of the present invention shows significant antitumor effect against 5 - FU resistant P 388 leukemia as ILS 40 - 45%. However, phospholipid conjugate of 2' - deoxy - 5 - fluorouridine wherein side chain is long chain acyl ( compound 8 ) described in Japanese Patent Unexam. Publ. No. 61 - 91195, is shown obviously no antitumor activity against 5 - FU resistant P 388 leukemia.

Table 1 Antitumor effect against Meth A fibrosarcoma ( sc - po )

| Compound No | Dose m mol/kg/dai | Total Dose m mol/kg/mice | T / C ( % ) 14 th day |
|---|---|---|---|
| 1 | 0. 1 | 0. 5 | 23. 8** |
| 2 | 0. 1 | 0. 5 | 37. 3* |
| 3 | 0. 1 | 0. 5 | 32. 1** |
| 4 | 0. 1 | 0. 5 | 29. 8** |
| 5 | 0. 1 | 0. 5 | 22. 2** |
| 6 | 0. 1 | 0. 5 | 36. 3* |
| 7 | 0. 1 | 0. 5 | 26. 6** |
| A parent compound (2'-deoxy-5-trifluoromethyluridine) | 0. 1 | 0. 5 | 61. 2 |

N = 5

Significant differrence is checked by Student's t-test.

** $p < 0.01$ * $p < 0.05$

Table 2 Antitumor effect against 5 - FU resistant P 388 leukemia ( ip-ip )

| Compound No | Dose m mol/kg/day | Total Dose m mol/mice | I L S ( % ) |
|---|---|---|---|
| 1 | 0. 03 | 0. 15 | 45. 0 |
| 5 | 0. 03 | 0. 15 | 40. 0 |
| 8 | 0. 01 | 0. 05 | 11. 0 |
| 8 | 0. 03 | 0. 15 | −14. 0 |
| 5 F U | 0. 15 | 0. 75 | 8. 0 |

The following examples illustrate the present invention but are not to be construed as limiting.

Example 1

2' - deoxy - 5 - trifluoromethyluridine (237mg, 0.8mM) was dissolved in 0.2 M acetate buffer (pH 5.7, 20 ml) containing 0.25 M $CaCl_2$ (2 ml). PLDP ( Phospholipase D-P from genus Streptomyces, Toyo Jozo Co., specific activity 160 units/mg, 30mg ) and a solution of 1, 2 dipalmitoyl - sn - glycero - 3 - phosphocholine ( 1.76 g ) in chloroform ( 60 ml ) were added thereto, then the resultant mixture was stirred at 45 °C for 6 hours. To the reaction mixture were added Methanol ( 30 ml ) and water ( 13 ml ), and the resultant mixture was then partitioned. The organic layer was dried up in vacuo. The residue was purified by means of silica- gel flash column ( Merck Art 9385 silica-gel ) (solvent system; chloroform → chloroform : methanol= 20 : 1 → ditto 10 : 1 → ditto 4 : 1 ). The eluate fraction containing the product was dried up in vacuo and the resultant residue was dissolved in a mixture of chloroform and methanol ( 2 : 1 )( 40 ml ) and separated by adding 0.5 N HCl. The organic layer was washed twice with water, then dried up in vacuo. The residue was dissolved in a mixture of chloroform : methanol : water ( 10 : 5 : 1 ) ( 40 ml ), whereafter it was charged on a column of Diaion WK - 20 resin (Na type, Mitsubishi Chem. Co.) and eluted with the same solvent mixture. The eluate was recovered and dried up to obtain sodium salt of the product.

Yield: 477 mg

UV methanol $\lambda^{max}$ = 261 nm

MS (FAB) m/e = 949 ($MNa^+$)

Example 2

In example 1, 1, 2 - dipalmitoyl - sn - glycero - 3 - phosphocholine was replaced by glycerophospholipid of the formula [I] wherein $R_1$ and $R_2$ are illustrated in Table 3 to obtain the product② ~⑦ in Table 3.

Table 3

| Compound No | $R_1$ | $R_2$ | yield | UV methanol $\lambda^{max}$ | MS (FAB) |
|---|---|---|---|---|---|
| ② | stearoyl | stearoyl | 396mg | 261nm | m/e1005 ($MNa^+$) |
| ③ | oleoyl | oleoyl | 398mg | 261nm | m/e1001 ($MNa^+$) |
| ④ | palmitoyl | oleoyl | 401mg | 261nm | m/e 975 ($MNa^+$) |
| ⑤ | palmitoyl | linoleoyl | 293mg | 261nm | m/e 973 ($MNa^+$) |
| ⑥ | stearoyl | oleoyl | 421mg | 261nm | m/e1003 ($MNa^+$) |
| ⑦ | stearoyl | linoleoyl | 286mg | 261nm | m/e1001 ($MNa^+$) |

## Claims

1. A compound which is a nucleoside-phospholipid conjugate of formula:

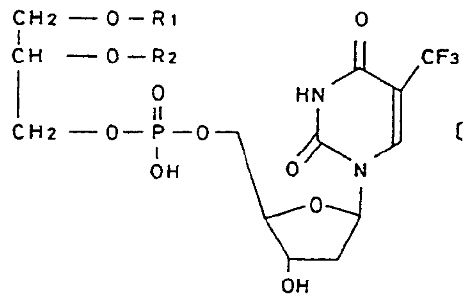

(I)

wherein $R_1$ and $R_2$, which are the same or different, are $C_{14-20}$ aliphatic acyl groups or a pharmacologically acceptable salt thereof.

2. A compound according to claim 1 wherein at least one of $R_1$ and $R_2$ is a $C_{16-18}$ aliphatic acyl group.

3. A compound according to claim 2 wherein $R_1$ is a palmitoyl, stearoyl or oleoyl group and $R_2$ is a palmitoyl, stearoyl, oleoyl or linoleoyl group.

4. A process for the preparation of a compound as defined in any one of claims 1 to 3, which comprises reacting a glycerophospholipid of formula (II):

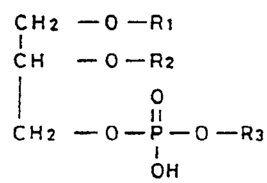

wherein $R_1$ and $R_2$ are as defined in claim 1 and $R_3$ is a choline residue, with a nucleoside in the presence of a phospholipase D and, if desired, converting the nucleoside-phospholipid conjugate thus obtained into a pharmacologically acceptable salt thereof.

5. A pharmaceutical composition which comprises a compound as defined in any one of claims 1 to 3 and a pharmaceutically acceptable diluent.

6. A compound as defined in any one of claims 1 to 3 for use in a method of treatment of the human or animal body by therapy.

7. A compound as defined in any one of claims 1 to 3 for use in a method of treatment of a tumor or leukemia.

8. Use of a compound as defined in any one of claims 1 to 3 in the manufacture of a medicament for the treatment of a tumor or leukemia.

European Patent Office

# EUROPEAN SEARCH REPORT

Application Number

EP 91304363.4

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl.5) |
| Y | EP - A2 - 0 350 287 (VICAL, INC.) * Claims 1,2,27,29,32,34,35, 58,61,63 * -- | 1-3,5-8 | C 07 H 19/10<br>C 07 H 19/073<br>A 61 K 31/70<br>A 61 K 47/48<br>C 12 P 19/30 |
| D,Y | CANCER RESEARCH, vol 24, December 1964 C. HEIDELBERGER, S.W. ANDERSON "Fluorinated Pyrimidines. XXI. The Tumor-inhibitory Activity of 5-Trifluoromethyl-2'-deoxyuridine" pages 1979-1985 * Page 1979; summary * -- | 1-3,5-8 | |
| A | US - A - 4 921 951 (SATOSHI SHUTO et al.) * Abstract; column 1, line 65 - column 3, line 21 * -- | 1-8 | |
| D,A | US - A - 4 797 479 (SATOSHI SHUTO et al.) * Abstract; claims 1,2,4 * -- | 1-8 | TECHNICAL FIELDS SEARCHED (Int. Cl.5) |
| D,A | PATENT ABSTRACTS OF JAPAN, unexamined applications, C field, vol. 10, no. 269, September 12, 1986 THE PATENT OFFICE JAPANESE GOVERNMENT page 37 C 372 * Kokai-no. 61-91 195 (TOYAMA CHEM CO LTD) * -- | 1-3,5-8 | C 07 H 19/00<br>A 61 K 31/00<br>A 61 K 47/00 |
| A | EP - A2 - 0 122 151 (MEITO SANGYO KABUSHIKI KAISHA) * Claims 1-3,5; page 38, lines 4-34, table 2, compound no. 72 * | 1-6 | |

The present search report has been drawn up for all claims

| Place of search | Date of completion of the search | Examiner |
|---|---|---|
| VIENNA | 22-08-1991 | MAZZUCCO |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

European Patent Office

# EUROPEAN SEARCH REPORT

Application Number

-2-
EP 91304363.4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP - A2 - 0 103 783 (DR. THILO & CO. GMBH) * Claims 1,4,12; page 6, lines 15-28 * | 1-3,5, 6 | |
| | | | TECHNICAL FIELDS SEARCHED (Int. Cl.5) |

The present search report has been drawn up for all claims

| Place of search | Date of completion of the search | Examiner |
|---|---|---|
| VIENNA | 22-08-1991 | MAZZUCCO |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)